**Europäisches Patentamt**

(19) **European Patent Office**

**Office européen des brevets**

(11) Publication number: **0 012 008**
**B1**

(12) # EUROPEAN PATENT SPECIFICATION

(45) Date of publication of patent specification: **23.11.83**  (51) Int. Cl.³: **A 61 K 7/18**

(21) Application number: **79302706.1**

(22) Date of filing: **28.11.79**

(54) Toothpaste.

(30) Priority: **29.11.78 GB 4653078**

(43) Date of publication of application:
**11.06.80 Bulletin 80/12**

(45) Publication of the grant of the patent:
**23.11.83 Bulletin 83/47**

(84) Designated Contracting States:
**AT BE CH DE FR GB IT NL SE**

(56) References cited:
**GB - A - 1 132 830**
**GB - A - 1 292 263**
**US - A - 3 573 886**
**US - A - 3 966 863**
**US - A - 4 038 380**

**CHEMICAL ABSTRACTS, vol. 84, no. 8, 23rd
February 1976, page 388, no. 49742s
Columbus, Ohio, U.S.A.
CHEMICAL ABSTRACTS, vol. 72, no. 12, 23th
March 1970, page 292, no. 59085w Columbus,
Ohio, U.S.A.**

(73) Proprietor: **BEECHAM GROUP PLC
Beecham House Great West Road
Brentford Middlesex (GB)**

(72) Inventor: **Duke, Susan Ann
89 Holmbury Grove
Featherbed Lane Croydon, Surrey (GB)**

(74) Representative: **Cresswell, Thomas Anthony et al,
European Patent Attorney Beecham
Pharmaceuticals Great Burgh Yew Tree Bottom
Road
Epsom Surrey KT18 5XQ (GB)**

Courier Press, Leamington Spa, England.

# Toothpaste

This invention relates to oral hygiene compositions having anti-cariogenic activity, and more particularly to toothpastes, and mouthwashes.

It has been recognized for some years that the incorporation of ionic fluoride compounds into oral hygiene compositions such as toothpastes and mouthwashes provides them with anti-cariogenic activity. This activity is believed to result from the ability of fluoride to reduce the solubility of tooth enamel in slightly acid media which frequently occur in the mouth as a result of the action of bacteria on food. This solubility-reducing effect is thought to result from the conversion of hydroxyapatite, a principal mineral component of tooth enamel, into a less soluble mineral, fluoroapatite.

It is generally recognised that ionic monofluorophosphates are the most effective anti-cariogenic fluoridating agent. It is thought in some quarters that monofluorophosphates exert their effect at least in part, by hydrolysing to phosphate and fluoride ion, which is subsequently incorporated into tooth enamel.

It has been found that the presence of certain quantities of carbonate ion and metasilicate ion in a solution containing dissolved monofluorophosphate ion increases the quantity of free fluoride ion concentration available in solution and that when teeth are treated by such solutions, not only is there an increase in fluoride uptake over and above that which would be expected for the level of free fluoride present, but also there is a significant decrease in the dissolution rate of tooth enamel mineral after such treatment as compared with that expected for the level of free fluoride ions.

These effects are principally dependent upon the amount of dissolved metasilicate ion which is formed in the mouth, usually by dilution of an oral hygiene composition with saliva.

The effects are not seen in the presence of alumina.

Accordingly the present invention provides an oral hygiene composition, substantially free of alumina, comprising a source of metasilicate ions, a source of carbonate ions and a source of monofluorophosphate ions, the sources being present in amounts such that, in use, an aqueous solution containing from 3.5 to $7.0 \times 10^{-4}$ mol.l$^{-1}$ dissolved metasilicate ions, from 1.0 to $2 \times 10^{-2}$ mol.l$^{-1}$ dissolved monofluorophosphate ions and from $0.5 \times 10^{-4}$ to $1.0 \times 10^{-3}$ mol.l$^{-1}$ dissolved carbonate ions is produced in the mouth.

Preferably the concentration of metasilicate ions is about $5 \times 10^{-4}$ mol.l.

The source of metasilicate ions may be silicic acid or a physiologically acceptable salt thereof which is at least sparingly soluble in aqueous media. Examples of such salts include alkaline earth metal salts such as calcium and magnesium metasilicate. Calcium metasilicate is a particularly preferred source of ions.

The source of monofluorophosphate ions is suitably a physiologically acceptable salt which is soluble in aqueous media. Examples of such salts include alkali metal monofluorophosphates of which sodium monofluorophosphate $Na_2PO_3F$ is especially preferred.

The source of carbonate ions is also a soluble or sparingly soluble physiologically acceptable carbonate salt. Examples include alkali metal salts such as sodium and potassium carbonate and alkaline earth metal salts such as magnesium and calcium carbonate.

The preferred form of oral hygiene composition according to the invention is a toothpaste, which becomes extensively diluted by saliva in normal use. The degree of dilution in the mouth of a fluoride-containing toothpaste can be determined by the following standard test. A standard amount, viz 1 gm, of toothpaste containing a known amount of fluoride is dispensed onto a toothbrush. A volunteer uses this to brush his teeth for 30 seconds, and spits the contents of his mouth into a pre-weighed receiver. The weight of spittings can be measured and the concentration of fluoride in the spittings can be determined by estimating the total fluoride present.

In practice it is found that a toothpaste becomes diluted about four fold by saliva when in use.

In general, when it is desired to apply a soluble active material in certain concentrations to teeth via a toothpaste, the paste is formulated four times more concentrated than the final concentration required in the mouth, to account for this dilution effect.

According to the invention, there is provided a toothpaste, substantially free of alumina, comprising from 1.5 to $3 \times 10^{-3}$ mol.l$^{-1}$ of dissolved metasilicate ions, from 4 to $8 \times 10^{-2}$ mol.l$^{-1}$ dissolved monofluorophosphate ions and from $2 \times 10^{-4}$ to $4 \times 10^{-3}$ mol.l$^{-1}$ dissolved carbonate ions in an aqueous toothpaste vehicle.

More suitably the composition contains not more than $6 \times 10^{-2}$ mol.l$^{-1}$ and preferably contains $5 \times 10^{-2}$ mol.l$^{-1}$ of dissolved monofluorophosphate ions.

The quantity of dissolved carbonate ions present in toothpaste according to this invention is generally not less than $2 \times 10^{-4}$ mol.l$^{-1}$ but is more suitably $5 \times 10^{-4}$ to $10 \times 10^{-4}$ mol.l$^{-1}$. Typically, a toothpaste formulation consists of an aqueous dispersion comprising an abrasive cleaning and/or polishing agent, a surfactant, a humectant, a thickener, and optionally flavouring and sweeting agents.

Typically, a toothpaste having the above concentrations of dissolved metasilicate, monofluorophosphate and carbonate ions comprise from 1.2 to 3.5% by weight of calcium metasilicate, from 0.6 to 1.2% by weight of an ionic monofluorophosphate, and from 10 to 60% by weight of calcium carbonate, in an aqueous toothpaste vehicle. The preferred weight range of calcium metasilicate is from 1.2 to 2.4%. Most preferably, the amount of calcium metasilicate in the toothpaste is 2% by weight.

With the exception of aluminas any conventional toothpaste abrasive may be used in accordance with this invention. Powdered forms of calcium carbonate in an abrasive form constitute one important class of such abrasives. Examples of these abrasives are milled limestone or marble, chalks such as aragonite, calcite or mixtures thereof, and synthetically precipitated chalks such as waterworks chalk. Generally, the calcium carbonate should have a weight median diameter of less than 40 microns, preferably less than 15 microns.

A second class of abrasives are powdered silicas, particularly, silica xerogels as defined in U.S. Patent No. 3,538,230.

Abrasive agents of the above two classes may be used alone or in admixture with each other or in admixture with other abrasive agents such as water insoluble sodium or potassium metaphosphates, hydrated or anhydrous dicalcium phosphate, calcium pyrophosphate, zirconium silicate or mixtures thereof.

Typically toothpaste formulations comprise 20 to 75% by weight of abrasive.

When the abrasive is a carbonate such as a chalk, it may also act as the carbonate ion source. However it may be desired to supplement the quantity of carbonate ion by adding a soluble carbonate salt such as sodium carbonate to the formulation. Of course where the abrasive is other than a carbonate abrasive, addition of a non-abrasive sparingly soluble or soluble carbonate is essential. The necessary quantity of dissolved carbonate ion present where a sparingly soluble carbonate is employed may be calculated from a knowledge of its solubility constant. However, it is generally more practical in view of the number of constituents which a toothpaste contains to make a four fold dilution of the formulation with water and to measure the amount of dissolved carbonate in that solution.

The surfactant is normally a water-soluble non-soap or synthetic organic detergent. Suitable surfactants include the water-soluble salts of: higher fatty acid monoglyceride monosulphates (for example sodium hydrogenated coconut fatty acid monoglyceride monosulphate); higher alkyl sulphates (for example sodium lauryl sulphate); alkylarylsulphonates (for example sodium dodecylbenzenesulphonates); and higher alkyl sulphoacetates (for example sodium lauryl sulphoacetate). There may also be used the saturated higher aliphatic acyl amides of lower aliphatic amino carboxylic acids having 12 to 16 carbon atoms in the acyl radical and in which the amino acid portion is derived from the lower aliphatic saturated monoaminocarboxylic acids having 2 to 6 carbon atoms, such as the fatty acid amides of glycine, sarcosine, alanine, 3-aminopropanoic acid and valine, particularly the N-lauroyl, myristoyl and palmitoyl sarcosinate compounds. Conventional non-ionic surfactants may also be included if desired.

The surface-active materials are generally present in an amount of 0.05 to 10%, preferably 0.5 to 5% by weight of the composition.

Typical humectants which may be used in toothpastes in accordance with this invention include glycerine, sorbitol and propylene glycol, either alone or in admixture with each other. It is preferred to use mixtures of sorbitol and glycerine. Typically, the total liquid content of a toothpaste, that is to say, water and humectant is 20 to 75% by weight of the preparation.

Typical thickening agents which may be used in toothpastes in accordance with this invention include natural and synthetic gums, and gum-like materials such as Irish Moss, gum tragacanth, sodium carboxymethylcellulose, polyvinylpyrrolidone, or starch. Irish Moss and sodium carboxymethylcellulose are preferred. Generally the gum content is less than 10% by weight of the formulation and is preferably 0.5 to 5% by weight.

Materials which toothpaste formulations according to the invention may optionally contain are sweeteners such as saccharin; flavouring agents such as oils of spearmint, wintergreen or peppermint, chloroform, colouring or whitening agents such as titanium dioxide; preservatives such as sodium benzoate; emulsifying agents; acidifying agents such as citric acid; silicones; alcohol; methanol; chlorophyll compounds such as sodium copper chlorophyllin, and anti-bacterial agents such as chlorhexidine.

If desired, the toothpaste may be formulated as cored or striped toothpaste compositions, particularly with an opaque core or stripes (containing the calcium carbonate abrasive and the metasilicate ingredient) within or on a transparent gel. The gel can contain the monofluorophosphate ingredient and in such cases the composition is taken as a whole in determining proportions of ingredients as aforesaid.

The pH of toothpastes according to this invention is mildly alkaline, that is to say, the pH is between 8.5 and 10.5.

Toothpastes in accordance with this invention are prepared in the usual way.

The invention is now described in more detail with reference to the following examples and accompanying drawing, which is a graph showing the effect of increasing calcium metasilicate concentration in a toothpaste composition on the fluorohydroxyapatite formation in tooth enamel.

**0 012 008**

### Example 1
A toothpaste having the following formulation was made up:

|  | % W/W |
| --- | --- |
| Glycerin | 15.63 |
| Sorbitol (70% solution) | 10.40 |
| Carboxymethyl cellulose gum | 0.75 |
| Hydroxyethyl cellulose gum | 0.07 |
| Sodium saccharin (15% solution) | 1.00 |
| Sodium monofluorophosphate | 0.80 $(5 \times 10^{-2}$ M) |
| Chalk abrasive | 44.77 (4.5 M) |
| Sodium lauryl sulphate | 1.77 |
| Flavour ingredients | 1.00 |
| Calcium metasilicate | 2.00 $(1.5 \times 10^{-1}$ M) |
| Water to | 100.00 |

A mucilage was first prepared by blending the carboxymethyl cellulose, hydroxyethyl cellulose and calcium metasilicate. The mixture, together with the saccharin, glycerin and sorbitol, was placed in a suitable mixing vessel fitted with a high shear mixer, and mixing was carried out until a homogeneous distribution was obtained. Water, pre-heated to 55°C, was then added followed by sodium monofluorophosphate, and mixing was continued. The mixture was allowed to stand to permit the gums to hydrate. The flavour was then added and mixed in. The chalk abrasive was then added whilst under partial vacuum conditioning followed by the lauryl sulphate, and mixing was carried out until a homogeneous paste was obtained. Similar formulations having 0.2%, 0.5%, 1.0% and 5% calcium metasilicate were prepared, and the fluorohydroxyapatite formation (FHA) for each formulation was measured following a minute treatment of the enamel mineral, according to the method described in Caries Research Vol. 12, pages 12 to 20 (1978). The FHA values were plotted against the percentages by weight of calcium metasilicate in the toothpastes. The results are shown in the accompanying graph. The graphical results demonstrate that the most effective percentage range of calcium metasilicate in the toothpaste was from about 1.2 to 3.5% by weight, and the preferred amount was 2%.

4

Further examples are as follows:

Example 2
A striped toothpaste having the following formulations was made up:

| Clear Gel Phase | % W/W |
|---|---|
| Synthetic magnesium lithium silicate clay | 4.17 |
| Sorbitol (70% solution) | 60.00 |
| Polyethylene glycol 300 | 4.00 |
| Sodium carboxymethyl cellulose gum | 1.30 |
| Silica (colloidal) | 2.00 |
| Sodium monofluorophosphate | 0.80 |
| Saccharin | 0.15—0.25 |
| Flavour | 0.5—1.5 |
| Sodium lauryl sulphate | 1.5—2.5 |
| Water soluble dye | q.s |
| Water to | 100.00 |

| Opaque Paste Phase | % W/W |
|---|---|
| Sorbitol (70% solution) | 34.00 |
| Synthetic magnesium lithium silicate clay | 2.00 |
| Sodium carboxymethyl cellulose | 0.70 |
| Polyethylene glycol 300 | 2.00 |
| Titanium dioxide | 0.50 |
| Flavour | 0.5—1.5 |
| Chalk abrasive | 38.84 (3.9 M) |
| Sodium monofluorophosphate | 0.80 ($5 \times 10^{-2}$ M) |
| Saccharin | 0.15—0.25 |
| Silica (colloidal) | 1.0 |
| Sodium lauryl sulphate | 1.5—2.5 |
| Calcium metasilicate | 3.16 ($2.4 \times 10^{-1}$ M) |
| Water to | 100.00 |

PREPARATION OF THE CLEAR GEL PHASE

Carboxymethyl cellulose was slurried with part of the polyethylene glycol 300 and this, together with the flavour and the silica, was slurried in part of the sorbitol in a vacuum mixing vessel. After mixing, the saccharin and sodium monofluorophosphate, each separately predissolved in water, were

# 0012008

added and the mixing was continued.

A dispersion of magnesium lithium silicate with part of the water, sorbitol and polyethylene glycol 300 was prepared and added to the vacuum mixing vessel. Mixing was carried out until a homogeneous clear gel was obtained.

PREPARATION OF THE OPAQUE PASTE PHASE

A mucilage was prepared by blending the magnesium lithium silicate clay with the water, part of the sorbitol and the titanium dioxide. The mucilage was transferred, with the flavour, to a suitable vacuum mixing vessel. After mixing under vacuum, the chalk abrasive and calcium metasilicate were added followed by the sodium monofluorophosphate and saccharin (each separately pre-dissolved in water), the silica slurried in part of the sorbitol and finally the sodium lauryl sulphate dissolved in water. Mixing was continued until a uniform paste was obtained. A striped product was obtained by combining the gel and paste phases using a tube filling machine.

Example 3

A striped toothpaste having the following formulation was made up:

| Clear Gel Phase | % W/W |
|---|---|
| polyethylene glycol 400 | 3.00 |
| Sodium carboxymethyl cellulose | 1.20 |
| Calcium carrageenan gum | 0.30 |
| Sorbitol (70% solution) | 59.64 |
| Saccharin | 0.05—0.10 |
| Calcium glycerophosphate | 0.21 |
| Sodium monofluorophosphate | 0.76 |
| Sodium benzoate | 0.20 |
| Sodium silicate solution | 0.20 |
| Water soluble dye(s) | q.s. |
| Precipitated silica | 15.00 |
| Flavour | 0.5—1.0 |
| Sodium lauryl sulphate | 1.0—1.5 |
| Water to | 100.0 |

6

**0 012 008**

| Opaque Paste Phase | % W/W |
|---|---|
| Polyethylene glycol 400 | 2.94 |
| Sodium carboxymethyl cellulose | 0.65 |
| Sodium monofluorophosphate | 0.76 ($5 \times 10^{-2}$ M) |
| Sorbitol (70% solution) | 35.82 |
| Saccharin | 0.05—0.10 |
| Sodium benzoate | 0.20 |
| Calcium metasilicate | 3.16 ($2.4 \times 10^{-1}$ M) |
| Calcium carbonate abrasive | 38.8 (3.9 M) |
| Titanium dioxide | 0.50 |
| Precipitated silica | 5.00 |
| Flavour | 0.5—1.0 |
| Sodium lauryl sulphate | 1.0—1.5 |
| Water to | 100.00 |

PREPARATION OF THE CLEAR GEL PHASE

The saccharin, sodium benzoate, sodium monofluorophosphate and the dye(s) were dissolved in a mixture of the water and part of the sorbitol. This solution was combined under vacuum with a dispersion of sodium carboxymethyl cellulose and calcium carrageenan in polyethylene glycol in a suitable vacuum mixing vessel. The precipitated silica, calcium glycerophosphate, flavour, and sodium lauryl sulphate were then added with separate mixing after each addition.

PREPARATION OF THE OPAQUE PASTE PHASE

The sodium monofluorophosphate, saccharin and sodium benzoate in the water and sorbitol were mixed in a suitable vacuum mixing vessel.

The sodium carboxymethyl cellulose, dispersed in polyethylene glycol was added and mixed under vacuum. The calcium carbonate, titanium dioxide, calcium metasilicate and precipitated silica were blended and added to the main mix and mixed under vacuum. The flavour and sodium lauryl sulphate were then added with mixing between each addition.

A striped product was obtained by combining the gel and paste phases using a tube filling machine.

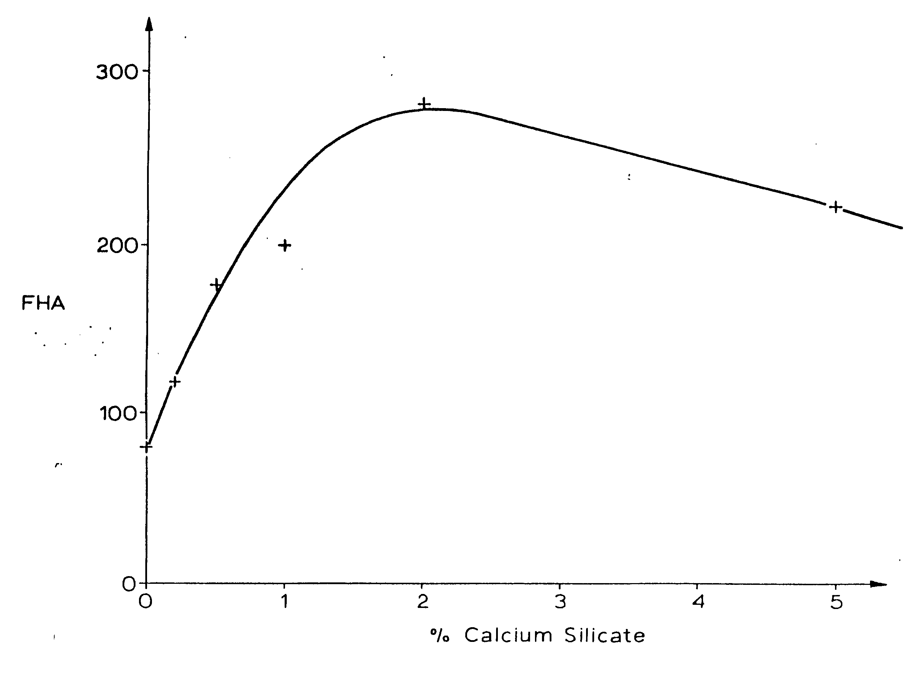

**Claims**

1. A toothpaste, substantially free of alumina, comprising a source of monofluorophosphate ions and a source of carbonate ions in an aqueous toothpaste vehicle, characterised in that a source of metasilicate ions is present, and the ionic concentrations of dissolved monofluorophosphate, carbonate and metasilicate ions are from 4 to $8 \times 10^{-2}$ mol.l$^{-1}$, from $2 \times 10^{-4}$ to $4 \times 10^{-3}$ mol.l$^{-1}$ and from 1.5 to $3 \times 10^{-3}$ mol.l$^{-1}$ respectively.

2. A toothpaste according to claim 1, characterised in that not more than $6 \times 10^{-2}$ mol.l$^{-1}$ of dissolved monofluorophosphate ions are present.

3. A toothpaste according to claim 1 or 2, characterised in that from $5 \times 10^{-4}$ to $10 \times 10^{-4}$ mol.l$^{-1}$ of dissolved carbonate ions are present.

4. A toothpaste according to claim 1, characterised in that it comprises from 1.2 to 3.5% by weight of calcium metasilicate, from 10 to 60% by weight of calcium carbonate, and from 0.6 to 1.2% by weight of an ionic monofluorophosphate, in an aqueous toothpaste vehicle.

5. A toothpaste according to claim 4 characterised in that it contains about 2% by weight of calcium metasilicate.

7

# 0 012 008

**Revendications**

1. Pâte dentifrice ne contenant sensiblement pas d'alumine, renfermant une source d'ions monofluorophosphate et une source d'ions carbonate dans un véhicule aqueux pour pâte dentrifrice, caractérisée en ce qu'une source d'ions métasilicate est présente et en ce que les concentrations ioniques en ions monofluorophosphate, carbonate et métasilicate en solution sont comprises entre 4 et $8 \times 10^{-2}$ moles.$\text{l}^{-1}$, entre $2 \times 10^{-4}$ et $4 \times 10^{-3}$ moles.$\text{l}^{-1}$ et entre 1,5 et $3 \times 10^{-3}$ moles.$\text{l}^{-1}$ respectivement.

2. Pâte dentrifrice suivant la revendication 1, caractérisée en ce que la quantité d'ions monofluorophosphate dissous présente n'est pas supérieure à $6 \times 10^{-2}$ moles.$\text{l}^{-1}$.

3. Pâte dentrifrice suivant la revendication 1 ou 2, caractérisée en ce que la quantité d'ions carbonate dissous présente est comprise entre $5 \times 10^{-4}$ et $10 \times 10^{-4}$ moles.$\text{l}^{-1}$.

4. Pâte dentrifrice suivant la revendication 1, caractérisée en ce qu'elle renferme de 1,2 à 3,5% en poids de métasilicate de calcium, de 10 à 60% en poids de carbonate de calcium et de 0,6 à 1,2% en poids d'un monofluorophosphate ionique, dans un véhicule aqueux pour pâte dentifrice.

5. Pâte dentrifrice suivant la revendication 4, caractérisée en ce qu'elle renferme environ 2% en poids de métasilicate de calcium.

**Patentansprüche**

1. Eine Zahnpaste, welche im wesentlichen frei ist von Aluminiumoxid und eine Quelle für Monofluorphosphationen und eine Quelle für Carbonationen in einer wäßrigen Zahnpastengrundsubstanz enthält, dadurch gekennzeichnet, daß eine Quelle für Metasilikationen vorhanden ist, und daß die Ionenkonzentrationen von gelösten Monofluorphosphat-, Carbonat- und Metasilikationen jeweils im Bereich von 4 bis $8 \times 10^{-2}$ mol.$\text{l}^{-1}$, von $2 \times 10^{-4}$ bis $4 \times 10^{-3}$ mol.$\text{l}^{-1}$ und von 1,5 bis $3 \times 10^{-3}$ mol.$\text{l}^{-1}$ liegen.

2. Eine Zahnpaste gemäß Anspruch 1, dadurch gekennzeichnet, daß nicht mehr als $6 \times 10^{-2}$ mol.$\text{l}^{-1}$ an gelösten Monofluorphosphationen vorhanden sind.

3. Eine Zahnpaste gemäß Anspruch 1 oder 2, dadurch gekennzeichnet, daß von $5 \times 10^{-4}$ bis $10 \times 10^{-4}$ mol.$\text{l}^{-1}$ an gelösten Carbonationen vorhanden sind.

4. Eine Zahnpaste gemäß Anspruch 1, dadurch gekennzeichnet, daß sie von 1,2 bis 3,5 Gewichtsprozent Calciummetasilikat, von 10 bis 60 Gewichtsprozent Calciumcarbonat und von 0,6 bis 1,2 Gewichtsprozent eines ionischen Monofluorphosphats in einer wäßrigen Zahnpastengrundsubstanz enthält.

5. Eine Zahnpaste gemäß Anspruch 4, dadurch gekennzeichnet, daß sie ungefähr 2 Gewichtsprozent Calciummetasilikat enthält.

FHA

% Calcium Silicate

0 0 1 2 0 0 8